# EUROPÄISCHE PATENTSCHRIFT

(11) **EP 0 462 311 B1**
(45) Veröffentlichungstag und Bekanntmachung des Hinweises auf die Patenterteilung: **05.04.1995**
(21) Anmeldenummer: 90111796.0
(22) Anmeldetag: 21.06.1990
(51) Int. Cl.: B06B 1/06

(54) **Verbund-Ultraschallwandler und Verfahren zur Herstellung eines strukturierten Bauelementes aus piezoelektrischer Keramik**
Composite ultrasound transducer and fabrication process of a structured component from piezoelectric ceramic
Transducteur ultrason composite et procédé de fabrication d'un élément structurel d'une céramique piézoélectrique

(43) Veröffentlichungstag der Anmeldung: 27.12.1991
(73) Patentinhaber: SIEMENS AKTIENGESELLSCHAFT, 80333 München (DE)
(72) Erfinder: Bast, Ulrich, Dr., D-8000 München 40 (DE); Vogt, Martina, D-8510 Fürth (DE); Kaarmann, Hans, Dr., D-8520 Buckenhof (DE); Wersing, Wolfram, D-8048 Kirchheim (DE); Lubitz, Karl, Dr., D-8012 Ottobrunn (DE); Cramer, Dieter, D-8150 Holzkirchen (DE)

(56) Entgegenhaltungen:
- EP-A- 0 006 623
- EP-A- 0 189 520
- DE-A- 3 739 226
- FR-A- 2 400 814
- GB-A- 2 114 856

## Beschreibung

Die Erfindung betrifft einen Verbund-Ultraschallwandler mit im wesentlichen in Längsrichtung abstrahlenden Wandlerelementen aus piezoelektrischer Keramik sowie ein Verfahren zur Herstellung eines Bauelementes, das mindestens eine strukturierte Komponente aus piezoelektrischer Keramik enthält.

Verbund-Ultraschallwandler, auch Composite-Ultraschallwandler genannt, werden in der medizinischen Diagnostik eingesetzt. Ein Verbund-Ultraschallwandler ist aus vielen kleinen piezoelektrisch aktiven, einzelnen Wandlerelementen aufgebaut. Die Wandlerelemente sind in ihren Abmessungen so konzipiert, daß sie im wesentlichen in Längsrichtung abstrahlen. Die Wandlerelemente eines Verbund-Ultraschallwandlers sind in eine Kunststoffmatrix so eingelagert, daß die Längsrichtungen der Wandlerelemente zueinander parallel sind. Die Länge der einzelnen Wandlerelemente bestimmt die Dicke des Verbund-Ultraschallwandlers.

Der Verbund-Ultraschallwandler stellt einen strukturierten Ultraschallwandler dar. Strukturierte Ultraschallwandler haben gegenüber unstrukturierten viele Vorteile. Durch die Unterteilung in einzelne Wandlerelemente werden niederfrequente Quermoden des Ultraschallwandlers, die zu Artefakten im Bild führen, vermindert oder unterdrückt. Durch die Strukturierung verringert sich die Klemmung der Wandlerelemente. Infolgedessen steigen Kopplungsfaktor, Piezomodul und damit Schallintensität des Verbund-Ultraschallwandlers in Dickenrichtung und nähern sich den höheren Werten eines in Längsrichtung schwingenden Stabes an. Die einzelnen Wandlerelemente können gruppenweise durch entsprechend unterteilte Elektroden elektrisch angesteuert werden. Dadurch wird die Richtung oder Fokussierung des Ultraschallsignals elektrisch veränderbar. Bei Phased-array-Wandlern oder Annular-Array-Wandlern findet dieses Anwendung.

Die Eigenschaften des Verbund-Ultraschallwandlers sind von der Form, Größe und Anordnung der einzelnen Wandlerelemente abhängig.

Aus der Patentschrift DE 3437862 C2 ist ein Verbund-Ultraschallwandler bekannt, bei dem als Wandlerelemente quadratische Prismen aus piezoelektrischer Keramik in regelmäßiger, linearer Anordnung in eine Polymermatrix eingebettet sind. Die Herstellung eines solchen Verbund-Ultraschallwandlers erfolgt nach einer Säge- und Fülltechnik (auch Dice- and fill-Technik genannt). In dieser Technik wird eine gesinterte keramische Scheibe durch Kreuz- und Quersägen in Prismen unterteilt. Die Sägeschnitte haben eine geringere Tiefe als es der Dicke der Keramikscheibe entspricht, so daß ein zusammenhängender Keramikboden stehen bleibt. Die Sägeschnitte werden mit Kunststoff gefüllt. Dann wird der Keramikboden abgeschliffen.

Bedingt durch die Herstellungstechnik sind Geometrie und Anordnung der Einzelelemente des Ultraschallwandlers Einschränkungen unterworfen. Die erreichbare Feinheit der Sägeschnitte ist durch die Dicke des Sägeblattes begrenzt. Dadurch ist die Herstellung von Ultraschallwandlern mit Arbeitsfrequenzen größer als 7,5 MHz nur eingeschränkt möglich. Durch das Sägen können nur gerade Schnitte mit senkrechten Flanken erzeugt werden. Die Seitenflächen der durch Sägen strukturierten einzelnen Wandlerelemente sind daher parallel. Parallele Seitenflächen der Wandlerelemente begünstigen jedoch das Ausbilden von unerwünschten Quermoden. Bedingt durch die geraden, über die gesamte Keramikscheibe verlaufenden Schnitte werden in dieser Technik sehr regelmäßige Anordnungen insbesondere mit unvertretbar großen Abständen zwischen den einzelnen Wandlerelementen erzeugt. Die großen Abstände führen zu einer Erniedrigung des Anteils der schallaktiven Keramikfläche.

Aus der DE-A-37 39 226 ist ein Ultraschallwandlerarray bekannt, bei dem die Trennfugen zwischen den einzelnen Wandlerelementen durch mehrere unterschiedlich breite Sägeschnitte hergestellt werden, so daß die Trennfugen Querschnitte aufweisen, die stufenförmig an ein Trapez angenähert sind.

Feinsagen ist ein sehr zeitaufwendiges Verfahren. Die Gefahr von Beschädigungen, wie zum Beispiel Ausbrüchen beim sägen, ist besonders groß bei feinen Strukturen, wie sie für Verbund-Ultraschallwandler mit hohen Arbeitsfrequenzen erforderlich sind. Das Produkt erreicht beim Sägen bereits eine hohe Veredelungsstufe, so daß Beschädigungen oder Zerstörungen beim Sägen besonders schwerwiegend sind.

Der Erfindung liegt die Aufgabe zugrunde, einen Verbund-Ultraschallwandler anzugeben, bei dem aufgrund seiner Geometrie die Ausbildung von Quermoden stärker unterdrückt ist als im Stand der Technik. Es ist ferner Aufgabe, ein Herstellverfahren anzugeben, mit dem ein solcher Verbund-Ultraschallwandler möglichst ohne Einschränkungen an sein Design herstellbar ist.

Die Aufgabe wird erfindungsgemäß gelost durch einen Verbund-Ultraschallwandler mit im wesentlichen in Längsrichtung abstrahlenden Wandlerelementen aus piezoelektrischer Keramik mit folgenden Merkmalen:
a) die Wandlerelemente sind in einer Ebene senkrecht zu ihrer Längsrichtung in einer Kunststoffmatrix eingelagert, so daß zwischen den Wandlerelementen mit Kunststoff vollständig gefüllte Zwischenräume bestehen,
b) die Wandlerelemente weisen eine solche geometrische Struktur auf und sind so zueinander angeordnet, daß eine Ausbildung von Schwingungsmoden senkrecht zur Längsrichtung der Wandlerelemente in dem Verbund-Ultraschallwandler unterdrückt wird,
c) die Wandlerelemente weisen in einer Schnittebene, die ihre Längsrichtung enthält, einen trapezförmigen Querschnitt auf,
d) die Wandlerelemente sind so angeordnet, daß es senkrecht zur ihrer Längsrichtung keine über den ganzen Verbundultraschallwandler geradlinig durchgehenden Kunststoffkanäle gibt.

Es liegt im Rahmen der Erfindung, die Ausbildung von Schwingungsmoden in Querrichtung dadurch zu unterdrucken, daß die Wandlerelemente in einer Schnittebene, die ihre Längsrichtung enthält, einen trapezförmigen Querschnitt aufweisen. Durch den trapezförmigen Querschnitt sind die Seitenflächen der Wandlerelemente in Längsrichtung nicht parallel. Dadurch können sich in diesem Fall weniger Querwellen ausbilden. Ferner wird durch den trapezförmigen Querschnitt die Gefahr des mechanischen Übersprechens zwischen benachbarten Elementen reduziert.

Es liegt ferner im Rahmen der Erfindung, die Wandlerelemente in unregelmäßiger, nicht linearer Weise anzuordnen. Dabei ist sowohl eine statistische als auch eine fluktuierende Größenverteilung der Wandlerelemente möglich. Bei einer flukturierenden Größenverteilung ist der Verbund-Ultraschallwandler in Bereiche unterteilt, wobei einem Bereich angehörende Wandlerelemente gleich groß sind, die Wandlerelemente verschiedener Bereiche jedoch unterschiedlich groß sind. Dadurch wird gegenüber regelmäßigen, rechtwinkligen Gitteranordnungen die Ausbildung störender Schwingungsmoden in Querrichtung weiter erschwert. Darüberhinaus hat diese Ausführungsform technologische Vorteile. Es wird die Gefahr vermindert, daß Ablösungen zwischen den Wandlerelementen und dazwischengefülltem Kunststoff auftreten infolge unterschiedlicher Ausdehnungskoeffizienten oder bei einer mechanischen Bearbeitung.

Durch Ausbildung der Wandlerelemente mit einem senkrecht zu ihrer Längsrichtung sechseckigen Querschnitt ist eine hohe Packungsdichte der Wandlerelemente im Verbund-Ultraschallwandler erzielbar. Die nutzbare Wandlerfläche wird dadurch vergrößert.

Zur Unterdrückung von unerwünschten kollektiven Schwingungsmoden im Verbund-Ultraschallwandler ist es vorteilhaft, wenn die Wandlerelemente senkrecht zu ihrer Längsrichtung unterschiedlich große Querschnitte aufweisen. Durch Einteilung der Wandlerelemente in Gruppen, wobei die Wandlerelemente einer Gruppe in einem räumlich zusammenhängenden Gebiet angeordnet sind und die Größe der Querschnitte senkrecht zu ihrer Längsachse um einen je Gruppe vorgegebenen Wert mit einer je Gruppe vorgegebenen Schwankungsbereich streuen, werden gezielt von der Geometrie des Verbund-Ultraschallwandlers abhängige Quermoden unterdrückt.

Die Verwendung von Wandlerelementen mit einem Aspektverhältnis (Höhe/Breite) im Bereich von 1,5 bis 2,0 ist vorteilhaft, da dadurch störende Schwingungsmoden in Querrichtung in einen leichter beherschbaren Frequenzbereich oberhalb der Nutzfrequenz, das ist die Resonanz in Dickenrichtung, verlagert werden. Außerdem ist der Puls-Echo-Übertragungsfaktor bei diesem Verhältnis maximal.

Die Aufgabe wird ferner gelöst durch ein Verfahren zur Herstellung eines Bauelementes, das mindestens eine strukturierte Komponente aus piezoelektrischer Keramik enthält, mit folgenden Schritten:
a) es wird eine Form verwendet, die ein Negativ einer vorgegebenen Struktur für die Komponente darstellt,
b) es wird ein Keramikschlicker in die Form gegossen, aus dem durch Trocknen und Brennen die Komponente aus piezoelektrischer Keramik entsteht,
c) die Form wird aus einem Kunststoff erzeugt, der beim Brennen ohne feste Rückstände quantitativ verbrennt, ohne die beim Trocknen fixierte Struktur der Komponente zu verändern.

Die Struktur der fertigen Komponente aus piezoelektrischer Keramik wird im wesentlichen durch die Form festgelegt. Einschränkungen an das Design sind daher nur durch die Möglichkeiten zur Erzeugung der Form gegeben.

Es liegt im Rahmen der Erfindung, eine Form zu verwenden, die z. B. nach dem aus E.W. Becker et al, Microelectronic Engineering, Band 4, (1986Y) S. 35 ff bekannten LIGA-Verfahren hergestellt ist. Dabei wird die Struktur der Form tiefenlithographisch definiert. Dabei ist jede Struktur realisierbar, die auf Papier gezeichnet werden kann. Es sind Strukturen mit Flanken einer vorgegebenen Neigung zur Senkrechten herstellbar.

In dieser Technik können Formen mit Strukturen mit beliebigen Querschnitt und beliebiger Anordnung hergestellt werden. Die Strukturen werden mit senkrechten oder schrägen Flanken, je nach Anwendung, hergestellt. Eine Einschränkung an die Form und die Anordnung der Strukturen stellt nur das Auflösungsvermögen der verwendeten Photolithographie dar.

Es ist vorteilhaft, die Bereitung des Keramikschlickers und das Füllen der Form mit dem Keramikschlicker unter Vakuum vorzunehmen, da dadurch Gasblasen im Keramikschlicker und Lufteinschlüsse in der Form vermieden werden.

Der Keramikschlicker muß mit einem Binder zubereitet werden, der die Form nicht anlöst. Es ist vorteilhaft, einen wasserlöslichen Binder zu verwenden, da dieser neben der Erfüllung der obigen Bedingung eine gute Ausbrennbarkeit aufweist und unter Umweltschutzgesichtspunkten unbedenklich ist.

Das Verfahren ist insbesondere geeignet zur Herstellung eines erfindungsgemäßen Verbund-Ultraschallwandlers. Dabei wird die Form so erzeugt, daß sie negative Strukturen entsprechend einer vorgegebenen Anordnung der Wandlerelemente vorgegebener Struktur darstellt und daß der Rand der Form die negativen Sturkturen überragt. Die Form wird mit dem Keramikschlicker bis über die negativen Strukturen gefüllt, so daß beim Trocknen und Brennen des Keramikschlickers ein zusammenhängender Boden aus piezoelektrischer Keramik entsteht. Hohlräume, die beim Brennen durch das Ausbrennen der Form entstehen, werden mit einem Werkstoff mit geringer mechanischer Kopplung und vernachlässigbarer elektrischer Leitfähigkeit gefüllt. Dieser unterdrückt durch seine dämpfenden Eigenschaften ein mechanisches Übersprechen von benachbarten Wandlerelementen im fertigen Verbund-Ultraschallwandler. Der zusammenhängende Boden wird am Schluß vollständig entfernt.

Dieses Verfahren hat den Vorteil, daß die Geometrie und Anordnung der Wandlerelemente auf die geplante Anwendung des Verbund-Ultraschallwandlers optimiert werden können. Einschränkungen für das Design des Verbund-Ultraschallwandlers sind nur durch das Auflösungsvermögen der bei der Strukturierung der Urform verwendeten Lithographie und durch die Notwendigkeit der vollständigen Auffüllung der negativen Strukturen in der Form mit dem Keramikschlicker gegeben. Einschränkungen ergeben sich daher nur im Bereich sehr kleiner Strukturen, nicht jedoch in Bezug auf die Anordnung und Form der Wandlerelemente. Untersuchungen haben gezeigt, daß Strukturen mit einem Durchmesser von 5 »m und mehr sicher herstellbar sind.

Weitere Ausgestaltungen der Erfindung gehen aus den übrigen Ansprüchen hervor.

Im folgenden wird die Erfindung anhand von Ausführungsbeispielen und der Figuren näher erläutert.
Fig. 1 zeigt in eine Kunststoffmatrix eingelagerte Wandlerelemente mit einem senkrecht zu ihrer Längsrichtung sechseckigen Querschnitt.
Fig. 2 zeigt einen Schnitt durch einen fertigen Verbund-Ultraschallwandler mit Wandlerelementen mit einem parallel zu ihrer Längsrichtung trapezförmigen Querschnitt.
Fig. 3 zeigt den mit III-III bezeichneten Querschnitt duch den Verbund-Ultraschallwandler aus Fig. 2.
Fig. 4 zeigt eine Draufsicht auf in eine Kunststoffmatrix eingelagerte Wandlerelemente mit unregelmäßigen, viereckigen Querschnitten.
Fig. 5 zeigt ein Ablaufdiagramm des erfindungsgemäßen Herstellverfahrens.

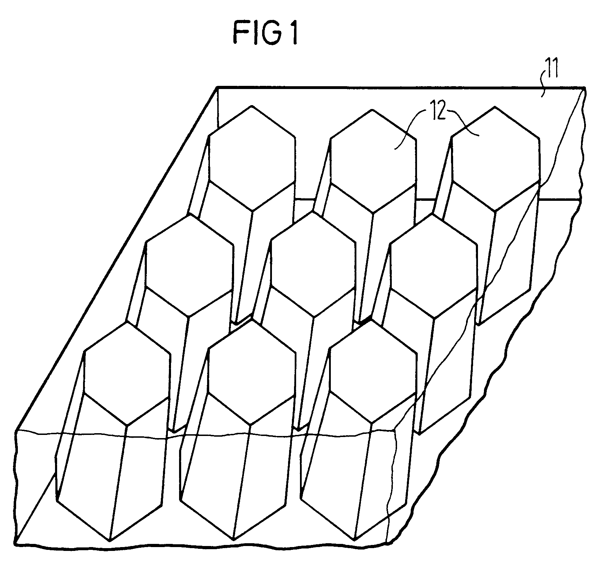

In Fig. 1 ist ein Ausschnitt einer Kunststoffmatrix 11 dargestellt. In die Kunststoffmatrix 11 sind Wandlerelemente 12 eingelagert. Die Kunststoffmatrix 11 besteht z. B. aus einem Polymer. Die Wandlerelemente 12 bestehen aus piezoelektrischer Keramik. Die Wandlerelemente 12 sind so angeordnet, daß ihre Längsrichtungen zueinander parallel sind. Die Wandlerelemente 12 weisen senkrecht zu ihrer Längsrichtung einen sechseckigen Querschnitt auf. Die Wandlerelemente 12 sind wabenförmig angeordnet, was zu einer guten Ausnutzung der verfügbaren Fläche führt. Parallel zu ihrer Längsrichtung weisen die Wandlerelemente einen trapezförmigen Querschnitt auf.

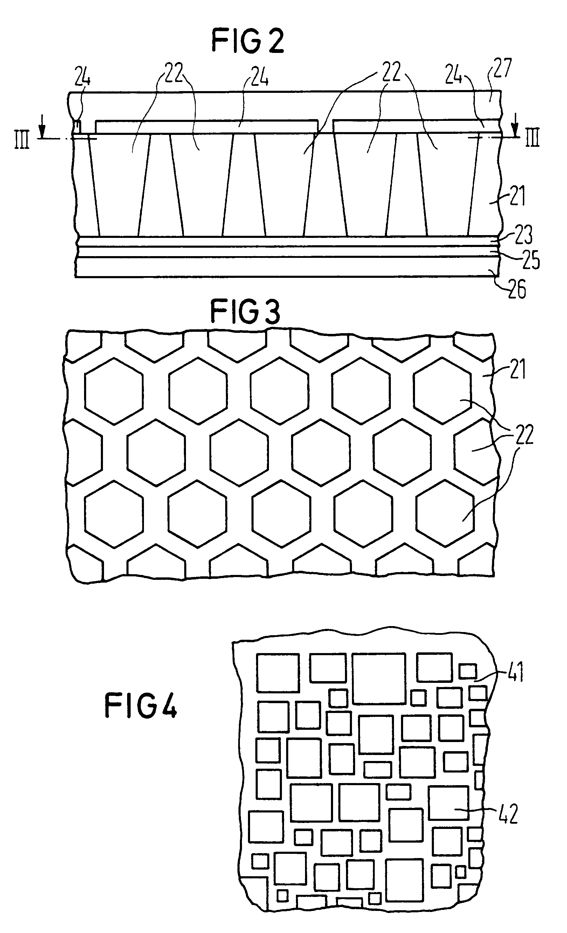

In Fig. 2 ist ein Schnitt durch einen fertigen Verbund-Ultraschallwandler parallel zur Längsrichtung der Wandlerelemente dargestellt. In einer Kunststoffmatrix 21 sind Wandlerelmente 22 so angeordnet, daß ihre Längsrichtungen zueinander parallel sind. Die Kunststoffmatrix 21 besteht aus einem Polymer. Die Wandlerelemente 22 bestehen aus piezoelektrischer Keramik, z. B. aus Bleizirkonattitanat. Die Wandlerelemente 22 haben parallel zu ihrer Längsrichtung einen trapezförmigen Querschnitt. Die Abweichungen der Seitenflächen von der Senkrechten betragen dabei z. B. 1 bis 5°. Die Grundfläche der Wandlerelemente 22 ist z. B. sechseckig (s. Fig. 3, die den Schnitt III-III der Fig. 2 darstellt). Die Wandlerelemente haben an der Grundlinie des trapezförmigen Querschnitts eine Kantenlänge z. B. im Bereich von 25 bis 350 »m. Die Höhe der Wandlerelemente 22 beträgt z. B. 50 bis 500 »m. Dabei muß ein Aspektverhältnis im Bereich 1,5 bis 2 eingehalten werden. Der Abstand zwischen benachbarten Wandlerelementen 22 beträgt an der Grundlinie des trapezförmigen Querschnitts z. B. 1 bis 50 »m.

In dem Verbund-Ultraschallwandler sind die Wandlerelemente 22 z. B. so angeordnet, daß die schmale Seite des trapezförmigen Querschnitts in Richtung der Senderichtung für den Ultraschall zeigt. Es ist ebenfalls möglich, die Wandlerelemente 22 so anzuordnen, daß die breite Seite des trapezförmigen Querschnitts in Senderichtung zeigt. Auf der Oberfläche der Kunststoffmatrix 21 und der Wandlerelemente 22, die in Senderichtung zeigt, ist eine durchgehende Elektrode 23 angeordnet. Auf der von der durchgehenden Elektrode 23 abgewandten Oberfläche der Kunststoffmatrix 21 und der Wandlerelemente 22 ist eine strukturierte Elektrode 24 angeordnet. Je nach Anwendung des Verbund-Ultraschallwandlers ist die strukturierte Elektrode 24 ringförmig oder linear strukturiert. Mit der strukturierten Elektrode 24 werden vorgegebene Wandlerelemente 22 zu getrennt ansteuerbaren Gruppen zusammengefaßt. Die durchgehende Elektrode 23 und die strukturierte Elektrode 24 bestehen z. B. aus gesputtertem CrPtAu.

Auf der durchgehenden Elektrode 23 sind in bekannter Weise eine oder mehrere Schichten zur Anpassung der akustischen Impedanz 25 angeordnet. Auf der Schicht zur Anpassung der akustischen Impedanz 25 ist eine fokussierende Linse 26 angeordnet. Die fokussierende Linse 26 kann entfallen, wenn sie in dem für den Verbund-Ultraschallwandler vorgesehenen Anwendungsfall nicht erforderlich ist. Auf der strukturierten Elektrode 24 ist in bekannter Weise eine Dämpfungsschicht 27 angeordnet. Die Dämpfungsschicht 27 absorbiert entgegen der Senderichtung abgegebenen Ultraschall.

In Fig. 4 ist eine weitere Ausführungsform eines erfindungsgemäßen Verbund-Ultraschallwandlers mit Wandlerelementen 42 dargestellt. Fig. 4 zeigt einen Schnitt senkrecht zur Längsrichtung der Wandlerelemente 42. Die Wandlerelemente 42 sind in einer Kunststoffmatrix 41 eingelagert. Die Wandlerelemente 42 zeigen einen viereckigen Querschnitt. Dabei variiert die Kantenlänge der einzelnen Wandlerelemente 42 innerhalb vorgegebener Grenzen. Die Wandlerelemente 42 sind so angeordnet, daß es senkrecht zur Längsrichtung der Wandlerelemente 42 keine über den ganzen Verbund-Ultraschallwandler durchgehende Kunststoffkanäle gibt. Durch die sich ergebende, unregelmäßige Anordnung der Wandlerelemente 42 werden über mehrere Wandlerelemente reichende unerwünschte Partialschwingungen unterdrückt. Dabei ist der Querschnitt der Wandlerelemente 42 parallel zu ihrer Längsrichtung z. B. rechteckig. Eine Verbesserung der Unterdrückung der Quermoden wird z. B. dadurch erzielt, daß der Querschnitt der Wandlerelemente 42 in Längsrichtung trapezförmig ist.

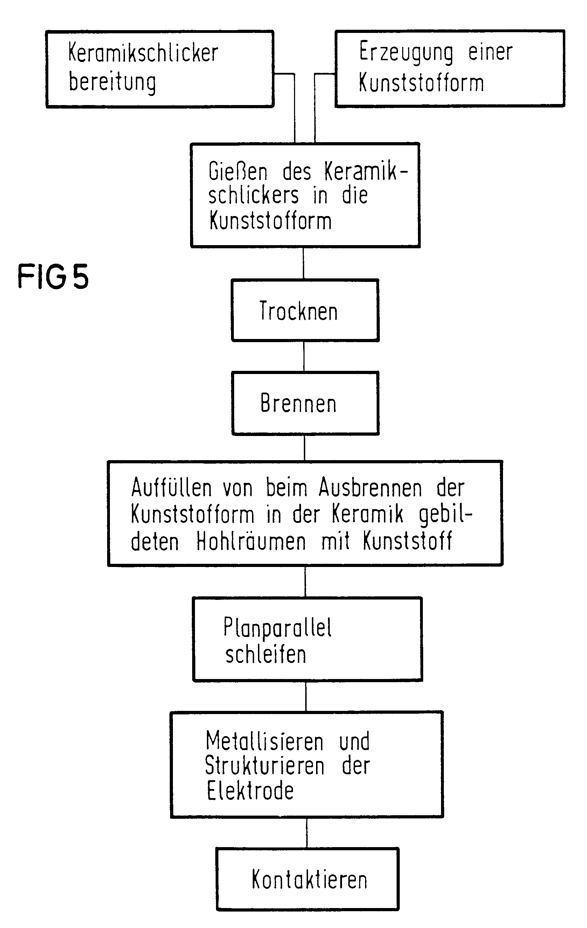

Anhand der Fig. 5 wird im folgenden das erfindungsgemäße Verfahren zur Herstellung eines Bauelementes, das mindestens eine strukturierte Komponente aus piezoelektrischer Keramik enthält, am Beispiel der Herstellung eines Verbund-Ultraschallwandlers näher erläutert.

Aus einem in konventioneller Weise hergestellten Keramikpulver, z. B. aus Bleizirkonattitanat, wird ein Schlicker angemacht. Der Schlicker wird in einer speziellen Füllvorrichtung unter Vakuum in eine Kunststofform gegossen, die das Negativ der gewünschten Struktur darstellt. Für diesen Verfahrensschritt werden verschiedene Forderungen an den Schlicker gestellt. Zum einen muß der Schlicker mit einem Bindemittel engemacht werden, das die Kunststofform nicht anlösen darf. Deshalb ist es vorteilhaft, den Schlicker mit einem wasserlöslichen Bindemittel wie z. B. Polyvinylalkohol anzumachen. Weitere Gründe für die Verwendung wasserlöslicher Bindemittel sind eine bessere Ausbrennbarkeit und der Umweltschutz. Eine weitere Forderung an den Schlicker ist eine zum Gießen geeignete Viskosität bei gleichzeitig hohem Litergewicht und Agglomeratfreiheit. Dieses wird durch Homogenisieren und Dispergieren des Schlickers, z. B. durch Mahlung des Schlickers in einer Attritormühle und Dispergieren in Ultraschall, erreicht.

Um Gasblasen im Schlicker und Lufteinschlüsse in der Gießform zu vermeiden, müssen sämtliche Verfahrensschritte vom Ansatz des Binders bis zum Füllen der Gießform unter Vakuum erfolgen.

Die strukturierte Kunststofform wird durch Abformung einer galvanoplastisch hergestellten, dazu negativen Form erzeugt. Eine bekannte Technik für die Herstellung der galvanoplastisch hergestellten Form ist z. B. das LIGA-Verfahren, das aus E.W. Becker et al, Microelectronic Engeneering, Bd. 4 (1986) S. 35 ff. bekannt ist. Die Kunststofform wird z. B. aus einem Reaktionsharz oder aus Thermoplast, z. B. Polymethylmethacrylat oder Polyoxymethylen, hergestellt.

Der Schlicker wird so hoch in die Kunststofform eingefüllt, daß die Strukturen der Kunststofform vollständig bedeckt sind und oberhalb der Strukturen der Kunststofform eine durchgehende Schicht des Schlickers ausgebildet wird. Aus der durchgehenden Schicht Keramikschlicker entsteht beim nachfolgenden Trocknen und Brennen ein zusammenhängender, unstrukturierter Bereich der piezoelektrischen Keramik, der als Träger für die Wandlerelemente dient.

Damit beim Trocknen des Keramikschlickers keine Risse entstehen, ist es vorteilhaft, die Trocknung durch ein definiertes Temperatur-/Feuchtigkeitsprogramm vorzunehmen. Der Keramikschlicker wird thermisch getrocknet. Da die Kunststofform beim Trocknen nicht schwindet, besteht die Gefahr, daß sich in der Keramik Sprünge bilden. Daher muß der Trocknungsprozeß sehr langsam und gleichförmig durchgeführt werden. Bis zu dem Punkt, an dem keine weitere Schwindung auftritt, ist es vorteilhaft, eine hohe relative Luftfeuchtigkeit von z. B. 90% und eine niedrige Temperatur von z. B. 30°C anzuwenden. Danach wird die Luftfeuchigkeit reduziert und die Temperatur erhöht.

Nach dem Trocknen wird der aus Kunststofform und getrockneter Keramikmasse bestehende Körper gebrannt. Beim Brand pyrolysieren die organischen Bestandteile. Für die Pyrolyse der organischen Bestandteile ist es vorteilhaft, ein spezielles Temperatur-/Atmosphärenprogramm zu verwenden. Für bestimmte Kunststoffe ist es insbesondere vorteilhaft, die Pyrolyse in einer reinen Sauerstoffatmosphäre durchzuführen. Bei der Pyrolyse verbrennt die Kunststofform quantitativ ohne feste Rückstände. Der Kunststoff für die Kunststofform wird so gewählt, daß der Kunststoff bei der Pyrolyse ohne Veränderung, insbesondere Beschädigung, der getrockneten Keramikstruktur verbrennt. Die Keramik sintert zu einem monolithischen Block, der die durch die Struktur der Kunststofform vorgegebenen Vertiefungen enthält. Die Vertiefungen werden mit einem Polymer vergoßen, das die Lage der Wandlerelemente fixiert, die mechanische Stabilität des Verbund-Ultraschallwandlers ergibt und die akustischen Anforderungen erfüllt. Insbesondere muß das Polymer Ausdehnungen und Kontraktionen der Wandlerelemente quer zu ihrer Längsrichtung aufnehmen. Zum Füllen der Vertiefungen wird ein Werkstoff mit geringer mechanischer Kopplung verwendet, damit nur geringes Übersprechen zwischen den Wandlerelementen des Verbund-Ultraschallwandlers auftritt. Für diesen Zweck sind insbesondere Epoxidharze und Acrylate geeignet. Anschließend werden die Deck- und Grundflächen des Verbund-Ultraschallwandlers planparallel geschliffen.

Es folgen bekannte Verfahrensschritte zur Fertigstellung des Verbund-Ultraschallwandlers. Auf die Ultraschall absendende Fläche wird eine ganzflächige Elektrode aufgebracht. Die Ultraschall absendende Fläche verläuft senkrecht zur Längsrichtung der Wandlerelemente. Auf die gegenüberliegende Seite wird eine strukturierte Elektrode aufgebracht. Die Elektroden werden z. B. durch Aufsputtern oder Aufdampfen von CrPtAu erzeugt. Das Strukturieren der strukturierten Elektrode erfolgt mittels herkömmlicher photolithographischer Verfahren. Auf die ganzflächige Elektrode werden in bekannter Weise eine oder mehrere Schichten zur Anpassung der akustischen Impedanz und, wenn es der Anwendungsfall erfordert, eine fokussierende Linse aufgebracht. Die ganzflächige Elektrode sowie die einzelnen Elemente der strukturierten Elektrode werden durch Anlöten oder Bonden dünner Drähte kontaktiert. Anschließend wird auf die strukturierte Elektrode ebenfalls in bekannter Weise eine Dämpfungsschicht aufgetragen.

Das erfindungsgemäße Verfahren hat den Vorteil, daß Form, Anordnung und Verteilung der einzelnen Wandlerelemente über die Verbund-Ultraschallwandlerfläche durch eine Maskenerstellung bestimmt werden. Damit können nicht lineare, z. B. flächendeckende Sechsecke, und auch bewußt unregelmäßige Strukturen in einfacher Weise erzeugt werden.

Das erfindungsgemäße Verfahren erlaubt die Erzeugung von definierten Querschnitten durch die Prozeßschritte Tiefenlithographie, Schlickerbefüllung und Sinterung. Der Querschnitt der Wandlerelemente wird dabei im Gegensatz zu bekannten Herstellverfahren nicht durch die Möglichkeiten der mechanischen Bearbeitung sondern durch die Erfordernisse an den fertigen Verbund-Ultraschallwandler festgelegt. Es sind insbesondere Wandlerelemente mit trapezförmigen Querschnitt herstellbar.

Da das Teil nach Schlickerguß und Trocknen gesintert wird, tragen die nach Vergießen mit Kunststoff im Inneren des Verbund-Ultraschallwandlers liegenden Wände der Wandlerelemente eine sogenannte Sinterhaut. Dies hat im Vergleich zu Wandlerelementen, die z. B. in der bekannten Sägetechnik hergestellt sind, den Vorteil einer mechanischen Defektfreiheit und ungestörter piezoelektrischer Eigenschaften in der Oberfläche. Die Rauhigkeit dieser Wände liegt im Bereich der Korngröße der Keramik. Diese Rauhigkeit sorgt für eine optimale Haftung des Polymers nach dem Verguß. Darüberhinaus läßt sich durch Beurteilung der Keramikoberflächen die Benutzung des vorliegenden Herstellverfahrens leicht nachweisen.

Das Herstellverfahren ist neben dem hier geschilderten Ausführungsbeispiel der Herstellung eines Verbund-Ultraschallwandlers ebenfalls zur Herstellung anderer, piezoelektrische Keramik enthaltenden Bauelemente, wie z. B. Biegeelemente und Antriebe, geeignet. Auch in diesen Anwendungsfällen macht sich vorteilhaft bemerkbar, daß die geometrische Form der aus piezoelektrischer Keramik bestehenden Komponenten nur durch das photolithographische Verfahren zur Herstellung der entsprechenden Kunststofform festgelegt wird.

## Patentansprüche

1. Verbund-Ultraschallwandler mit im wesentlichen in Längsrichtung abstrahlenden Wandlerelementen aus piezoelektrischer Keramik mit folgenden Merkmalen:
a) die Wandlerelemente (12, 22, 42) sind in einer Ebene senkrecht zu ihrer Längsrichtung in einer Kunststoffmatrix (11, 21, 41) eingelagert, so daß zwischen den Wandlerelementen (12, 22, 42) mit Kunststoff vollständig gefüllte Zwischenräume bestehen,
b) die Wandlerelemente (12, 22, 42) weisen eine solche geometrische Struktur auf und sind so zueinander angeordnet, daß eine Ausbildung von Schwingungsmoden senkrecht zur Längsrichtung der Wandlerelemente (12, 22, 42) in dem Verbund-Ultraschallwandler unterdrückt wird,
c) die Wandlerelemente (12, 22, 42) sind so angeordnet, daß es senkrecht zu ihrer Längsrichtung keine über den ganzen Verbund-Ultraschallwandler geradlinig durchgehenden Kunststoffkanäle gibt,
d) die Wandlerelemente (12, 22, 42) weisen in einer Schnittebene, die ihre Längsrichtung enthält, einen trapezförmigen Querschnitt auf.

2. Verbund-Ultraschallwandler nach Anspruch 1,
**dadurch gekennzeichnet,**
daß die Wandlerelemente (12, 22, 42) im wesentlichen dieselbe Länge aufweisen und an die Länge der Wandlerelemente (12, 22, 42) begrenzenden Flächen mit Elektroden (23, 24) versehen sind.

3. Verbund-Ultraschallwandler nach Anspruch 1 oder 2,
**dadurch gekennzeichnet,**
daß die Wandlerelemente (12, 22, 42) ein Aspektverhältnis zwischen 1,5 und 2 aufweisen.

4. Verbund-Ultraschallwandler nach einem der Ansprüche 1 bis 3,
**dadurch gekennzeichnet,**
daß die Wandlerelemente (12, 22, 42) in unregelmäßiger, nicht linearer Weise angeordnet sind.

5. Verbund-Ultraschallwandler nach einem der Ansprüche 1 bis 4,
**dadurch gekennzeichnet,**
daß die Wandlerelemente (12, 22, 42) Seitenflächen aufweisen, die um 1° bis 5° gegen ihre Längsrichtung verkippt sind.

6. Verbund-Ultraschallwandler nach einem der Ansprüche 1 bis 5,
**dadurch gekennzeichnet,**
daß die Wandlerelemente (12, 22, 42) in Längsrichtung eine Abmessung von 50 bis 500 »m haben und daß die Wandlerelemente (12, 22, 42) senkrecht zur Längsrichtung einen viereckigen oder sechseckigen Querschnitt mit einer Kantenlänge von 254 bis 350 »m an der Grundlinie des Trapezes aufweisen.

7. Verbund-Ultraschallwandler nach einem der Ansprüche 1 bis 6,
**dadurch gekennzeichnet,**
daß der Abstand benachbarter Wandlerelemente (12, 22) an der Grundlinie des trapezförmigen Querschnitts 1 bis 50 »m beträgt.

8. Verbund-Ultraschallwandler nach einem der Ansprüche 1 bis 4,
**dadurch gekennzeichnet,**
daß die Wandlerelemente (12, 22) senkrecht zu ihrer Längsrichtung einen sechseckigen Querschnitt aufweisen.

9. Verbund-Ultraschallwandler nach einem der Ansprüche 1 bis 8,
**dadurch gekennzeichnet,**
daß die Wandlerelemente (12, 22, 42) senkrecht zu ihrer Längsrichtung unterschiedlich große Querschnitte aufweisen.

10. Verbund-Ultraschallwandler nach Anspruch 9,
**dadurch gekennzeichnet,**
daß die Wandlerelemente (42) in Gruppen eingeteilt sind, wobei jedes Wandlerelement (42) genau einer Gruppe angehört, die Wandlerelemente (42) einer Gruppe in einem räumlich zusammenhängenden Gebiet angeordnet sind und die Größe der Querschnitte senkrecht zu ihrer Längsachse um einen je Gruppe vorgegebenen Wert mit einer je Gruppe vorgegebenen Schwankungsbreite streuen.

11. Verfahren zur Herstellung eines Bauelementes, das mindestens eine strukturierte Komponente aus piezoelektrischer Keramik enthält, mit folgenden Schritten:
a) es wird eine Form verwendet, die ein Negativ einer vorgegebenen Struktur für die Komponente darstellt,
b) es wird ein Keramikschlicker in die Form gegossen, aus dem durch Trocknen und Brennen die Komponente aus piezoelektrischer Keramik entsteht,
c) die Form wird aus einem Kunststoff erzeugt, der beim Brennen ohne feste Rückstände quantitativ verbrennt, ohne die beim Trocknen fixierte Struktur der Komponente zu verändern.

12. Verfahren zur Herstellung eines Verbund-Ultraschallwandlers, der in eine Kunststoffmatrix eingelagert, im wesentlichen in Längsrichtung abstrahlende Wandlerelemente aus piezoelektrischer Keramik enthält,
**gekennzeichnet durch** folgende Schritte:
a) eine Form wird so erzeugt, daß sie negative Strukturen entsprechend einer vorgegebenen Anordnung der Wandlerelemente vorgegebener Struktur enthält und daß der Rand der Form die negativen Strukturen überragt,
b) die Form wird mit einem Keramikschlicker, aus dem durch Trocknen und Brennen die Wandlerelemente entstehen, bis über die negativen Strukturen gefüllt, so daß beim Trocknen und Brennen des Keramikschlickers ein zusammenhängender, die Anordnung der Wandlerelemente fixierender Boden aus piezoelektrischer Keramik entsteht,
c) die Form wird aus einem Kunststoff erzeugt, der beim Brennen ohne feste Rückstände quantitativ verbrennt, ohne die beim Trocknen fixierte Anordnung und Struktur der Wandlerelemente zu verändern,
d) Hohlräume, die beim Brennen durch Ausbrennen der Form entstehen, werden mit einem Werkstoff mit geringer mechanischer Kopplung gefüllt, der durch seine dämpfenden Eigenschaften ein mechanisches Übersprechen von benachbarten Wandlerelementen im fertigen Verbund-Ultraschallwandler unterdrückt,
e) der zusammenhängende Boden wird vollständig entfernt.

13. Verfahren nach Anspruch 12,
**dadurch gekennzeichnet,**
daß der zusammenhängende Boden abgeschliffen wird.

14. Verfahren nach einem der Ansprüche 11 bis 13,
**dadurch gekennzeichnet,**
daß die Form aus einem der Werkstoffe Reaktionsharz oder Thermoplast erzeugt wird.

15. Verfahren nach einem der Ansprüche 11 bis 14,
**dadurch gekennzeichnet,**
daß die Bereitung des Keramikschlickers und das Füllen der Form mit dem Keramikschlicker unter Vakuum erfolgt.

16. Verfahren nach einem der Ansprüche 11 bis 15,
**dadurch gekennzeichnet,**
daß bei der Bereitung des Keramikschlickers ein wasserlösliches Bindemittel verwendet wird.

## Claims

1. Composite ultrasound transducer having transducer elements of piezoelectric ceramic which radiate substantially in the longitudinal direction, having the following features:
a) the transducer elements (12, 22, 42) are embedded in a polymer matrix (11, 21, 41) in a plane perpendicular to their longitudinal direction, so that between the transducer elements (12, 22, 42) there are interspaces completely filled with polymer,
b) the transducer elements (12, 22, 42) have such a geometrical structure and are arranged with respect to one another in such a way that any development of oscillation modes perpendicularly to the longitudinal direction of the transducer elements (12, 22 42) in the composite ultrasound transducer is suppressed,
c) the transducer elements (12, 22, 42) are arranged in such a way that there are no polymer channels rectilinearly extending completely across the entire composite ultrasound transducer perpendicularly to the longitudinal direction of the said transducer elements,
d) the transducer elements (12, 22, 42) have a trapezoidal cross-section in a sectional plane which contains their longitudinal direction.

2. Composite ultrasound transducer according to Claim 1, characterized in that the transducer elements (12, 22, 42) have substantially the same length and are provided with electrodes (23, 24) on faces bounding the length of the transducer elements (12, 22, 42).

3. Composite ultrasound transducer according to Claim 1 or 2, characterized in that the transducer elements (12, 22, 42) have an aspect ratio of between 1.5 and 2.

4. Composite ultrasound transducer according to one of Claims 1 to 3, characterized in that the transducer elements (12, 22, 42) are arranged in an irregular, non-linear way.

5. Composite ultrasound transducer according to one of Claims 1 to 4, characterized in that the transducer elements (12, 22, 42) have lateral faces which are tilted by 1° to 5° with respect to their longitudinal direction.

6. Composite ultrasound transducer according to one of Claims 1 to 5, characterized in that the transducer elements (12, 22, 42) have in the longitudinal direction a dimension of from 50 to 500 »m and in that the transducer elements (12, 22, 42) have perpendicularly to the longitudinal direction a rectangular or hexagonal cross-section with an edge length of from 254 to 350 »m at the baseline of the trapezium

7. Composite ultrasound transducer according to one of Claims 1 to 6, characterized in that the spacing of neighbouring transducer elements (12, 22) at the base line of the trapezoidal cross-section is 1 to 50 »m.

8. Composite ultrasound transducer according to one of Claims 1 to 4, characterized in that the transducer elements (12, 22) have a hexagonal cross-section perpendicularly to their longitudinal direction.

9. Composite ultrasound tranducer according to one of Claims 1 to 8, characterized in that the transducer elements (12, 22, 42) have cross-sections of varying size perpendicularly to their longitudinal direction.

10. Composite ultrasound transducer according to Claim 9, characterized in that the transducer elements (42) are divided into groups, each transducer element (42) belonging to precisely one group, the transducer elements (42) of one group being arranged in a spatially continuous region and the size of the cross-sections perpendicularly to their longitudinal axis varying by a value predetermined for each group with a range of divergence predetermined for each group.

11. Process for manufacturing a structural element which contains at least one structured component of piezoelectric ceramic, having the following steps:
a) a mould which represents a negative of a predetermined structure for the component is used,
b) a ceramic slip is poured into the mould, producing the component of piezoelectric ceramic by drying and baking
c) the mould is produced from a polymer which pyrolyses without solid residues during baking, without changing the structure of the component fixed during drying.

12. Process for manufacturing a composite ultrasound transducer which, embedded in a polymer matrix, contains transducer elements of piezoelectric ceramic radiating substantially in the longitudinal direction, characterized in that by the following steps:
a) a mould is produced in such a way that it contains negative structures corresponding to a predetermined arrangement of the transducer elements of a predetermined structure and in that the edge of the mould projects above the negative structures,
b) the mould is filled with a ceramic slip, producing the transducer elements by drying and baking, until over the negative structures, so that during drying and baking of the ceramic slip there is produced a continuous base of piezoelectric ceramic fixing the arrangement of the transducer elements,
c) the mould is produced from a polymer which pyrolyses without solid residues without baking, without changing the arrangement and structure of the transducer elements fixed during drying,
d) voids which occur during baking due to fritting of the mould are filled with a material having low mechanical properties, which by its damping characteristics suppresses mechanical crosstalking of neighbouring transducer elements in the finished composite ultrasound transducer,
e) the continuous base is removed completely.

13. Process according to Claim 12, characterized in that the continuous base is removed by grinding.

14. Process according to one of Claims 11 to 13, characterized in that the mould is produced from one of the materials, reactive resin or thermoplastic.

15. Process according to one of Claims 11 to 14, characterized in that the preparation of the ceramic slip and the filling of the mould with the ceramic slip takes place under a vacuum.

16. Process according to one of Claims 11 to 15, characterized in that a water-soluble binder is used in the preparation of the ceramic slip.

## Revendications

1. Transducteur à ultrasons composite comprenant des éléments transducteurs composites à ultrasons, qui émettent essentiellement dans la direction longitudinale et qui sont réalisés en une céramique piézoélectrique, présentant les caractéristiques suivantes :
a) les éléments transducteurs (12,22,42) sont insérés, dans un plan perpendiculaire à leur direction longitudinale, dans une matrice de matière plastique (11,21,41) de manière que des espaces intercalaires entièrement remplis de matière plastique existent entre les éléments transducteurs (12,22,42),
b) les éléments transducteurs (12,22,42) possèdent une structure géométrique telle et sont disposés les uns par rapport aux autres de telle manière que la formation de modes d'oscillations perpendiculaires à la direction longitudinale des éléments transducteurs (12,22,42) dans le transducteur composite à ultrasons, est supprimée,
c) des éléments transducteurs (12,22,42) sont disposés de telle manière qu'il n'existe, perpendiculairement à leur direction longitudinale, aucun canal en matière plastique s'étendant avec une forme rectiligne sur l'ensemble du transducteur composite à ultrasons,
d) les éléments transducteurs (12,22,42) possèdent une section transversale trapézoïdale ans un plan de coupe qui s'étend dans leur direction longitudinale.

2. Transducteur composite à ultrasons suivant la revendication 1, caractérisé par le fait que les éléments transducteurs (12,22,42) possèdent essentiellement la même longueur et sont pourvus d'électrodes (23,24), au niveau de surfaces qui délimitent la longueur des éléments transducteurs (12,22,42).

3. Transducteur composite à ultrasons suivant la revendication 1 ou 2, caractérisé par le fait que les éléments transducteurs (12,22,42) possèdent un taux d'élancement compris entre 1,5 et 2.

4. Transducteur composite à ultrasons suivant l'une des revendications 1 à 3, caractérisé par le fait que les éléments transducteurs (12,22,42) sont disposés d'une manière irrégulière et non linéaire.

5. Transducteur composite à ultrasons suivant l'une des revendications 1 à 4, caractérisé par le fait que les éléments transducteurs (12,22,42) possèdent des surfaces latérales qui sont inclinées de 1° à 5° par rapport à leur direction longitudinale.

6. Transducteur composite à ultrasons suivant l'une des revendications 1 à 5, caractérisé par le fait que les éléments transducteurs (12,22,42) possèdent une dimension comprise entre 50 et 500 »m dans leur dimension longitudinale et que les éléments transducteurs (12,22,42) possèdent, perpendiculairement à leur direction longitudinale, une section transversale quadrangulaire ou hexagonale avec une longueur de côté comprise entre 254 et 350 »m au niveau de la ligne de base du trapèze.

7. Transducteur composite à ultrasons suivant l'une des revendications 1 à 6, caractérisé par le fait que la distance d'éléments transducteurs voisins (12,22) au niveau de la ligne de base de la section transversale trapézoïdale est comprise entre 1 et 50 »m.

8. Transducteur composite à ultrasons suivant l'une des revendications 1 à 4, caractérisé par le fait que les éléments transducteurs (12,22) possèdent une section transversale hexagonale, perpendiculairement à leur direction longitudinale.

9. Transducteur composite à ultrasons suivant l'une des revendications 1 à 8, caractérisé par le fait que les éléments transducteurs (12,22,42) possèdent des sections transversales ayant des étendues différentes, perpendiculairement à leur direction longitudinale.

10. Transducteur composite à ultrasons suivant la revendication 9, caractérisé par le fait que les éléments transducteurs (42) sont répartis en groupes, chaque élément transducteur (42) appartenant précisément à un groupe, que les éléments transducteurs (46) d'un groupe sont disposés dans une région d'un seul tenant dans l'espace, et que les étendues des sections transversales perpendiculairement à leur axe longitudinal présentent une dispersion autour d'une valeur prédéterminée pour chaque groupe, avec une plage de variation prédéterminée pour chaque groupe.

11. Procédé pour fabriquer un composant qui comporte au moins un composant structuré formé d'une céramique piézoélectrique, au moyen des étapes suivantes :
a) on utilise un moule, qui représente un négatif d'une structure prédéterminée pour le composant,
b) on coule dans le moule une barbotine céramique, à partir de laquelle on obtient, par séchage et cuisson, le composant formé d'une céramique piézoélectrique,
c) on forme le moule avec une matière plastique qui, lors de la cuisson, cuit d'une manière quantitative sans déchets de résidus solides, sans modifier la structure du composant, fixée lors du séchage.

12. Procédé pour fabriquer un transducteur composite à ultrasons, qui comporte des éléments transducteurs qui sont insérés dans une matrice de matière plastique, émettent essentiellement dans la direction longitudinale et sont formés d'une céramique piézoélectrique, caractérisé par les étapes opératoires suivantes :
a) on réalise un moule de manière qu'il contienne des structures négatives correspondant à une disposition prédéterminée des éléments transducteurs d'une structure prédéterminée, et que le bord du moule fasse saillie par rapport aux structures négatives,
b) on remplit le moule avec une barbotine céramique, à partir de laquelle on obtient les éléments transducteurs par séchage et cuisson, jusqu'au-dessus des structures négatives de sorte que lors du séchage et de la cuisson de la barbotine céramique, on obtient un fond monobloc, qui fixe l'ensemble des éléments transducteurs, et est formé d'une céramique piézoélectrique,
c) on réalise le moule avec une matière plastique qui, lors de la cuisson, cuit d'une manière quantitative sans déchets de résidus solides, sans modifier la structure du composant, fixée lors du séchage,
d) on remplit les cavités, qui apparaissent lors de la cuisson par brûlage du moule, avec un matériau possédant un faible couplage mécanique et qui supprime, par ses caractéristiques d'amortissement, une diaphonie mécanique entre des éléments transducteurs voisins dans le transducteur composite à ultrasons à l'état terminé,
e) on élimine complètement le fond monobloc.

13. Procédé suivant la revendication 12, caractérisé par le fait qu'on élimine par meulage le fond monobloc.

14. Procédé suivant l'une des revendications 11 à 13, caractérisé par le fait qu'on réalise le moule avec l'un des matériaux : résine de réaction ou thermoplaste.

15. Procédé suivant l'une des revendications 11 à 14, caractérisé par le fait que la préparation de la barbotine céramique et le remplissage du moule avec la barbotine céramique s'effectuent sous vide.

16. Procédé suivant l'une des revendications 11 à 15, caractérisé par le fait que lors de la préparation de la barbotine céramique, on utilise un liant soluble à l'eau.
